(19) Europäisches Patentamt
European Patent Office
Office européen des brevets

(11) EP 0 612 716 B1

(12) **FASCICULE DE BREVET EUROPEEN**

(45) Date de publication et mention de la délivrance du brevet:
**08.05.1996 Bulletin 1996/19**

(51) Int. Cl.[6]: **C07C 215/28**, C07C 255/41

(21) Numéro de dépôt: **94400409.2**

(22) Date de dépôt: **25.02.1994**

(54) **Procédé pour la préparation d'un aminoalcool optiquement pur**

Verfahren zur Herstellung von einem optisch reinen Aminoalkohol

Process for the preparation of an optically pure aminoalcohol

(84) Etats contractants désignés:
**AT BE CH DE DK ES FR GB GR IE IT LI LU MC NL PT SE**

(30) Priorité: **26.02.1993 FR 9302262**

(43) Date de publication de la demande:
**31.08.1994 Bulletin 1994/35**

(73) Titulaire: **SANOFI**
**F-75008 Paris (FR)**

(72) Inventeurs:
- **Descamps, Marcel**
**F-31600 Lherm (FR)**
- **Radisson, Joel**
**F-31600 Saubens (FR)**

(74) Mandataire: **Gillard, Marie-Louise et al**
**Cabinet Beau de Loménie**
**158, rue de l'Université**
**F-75340 Paris Cédex 07 (FR)**

(56) Documents cités:
**EP-A- 0 428 434**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 612 716 B1

## Description

La présente invention concerne un procédé pour la préparation de la 2-(3,4-dichlorophényl)-4-hydroxybutylamine dextrogyre de formule :

(+) 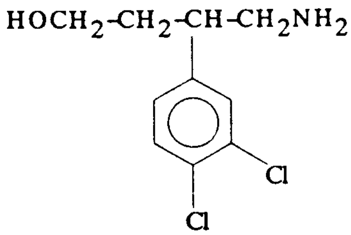

(I)

Le composé (I), décrit dans les demandes EP 0 428 434, 0 474 561, 0 515 240, 0 474 561 et 0 559 538 constitue un intermédiaire clé dans la synthèse d'arylalkylamines optiquement pures, thérapeutiquement actives en tant qu' antagonistes de tachykinines.

Selon les documents ci-dessus, le composé (I) est préparé par résolution du racémate via son D-(-)tartrate.

On a maintenant trouvé que par traitement de l'acide 3-cyano-3-(3,4-dichlorophényl)propionique racémique avec la D-(-)-N-méthylglucamine, on effectue une transformation asymétrique de second ordre conduisant à l'acide (-)-3-cyano-3-(3,4-dichlorophényl)propionique qui, par réduction énantioconservatrice avec un borane, donne le composé (I).

Il a été également trouvé de façon surprenante que le composé de formule (I) peut être obtenu à partir du 3,4-dichlorophénylacétonitrile par réaction avec un haloacétate alcalin, de préférence le chloroacétate de sodium, dédoublement *in situ* de l'acide 3-cyano-3-(3,4-dichlorophényl)propionique et réduction énantioconservatrice comme indiqué ci-dessus.

Ainsi, selon un de ses aspects, la présente invention concerne un procédé pour la préparation de la (+)-2-(3,4-dichlorophényl)-4-hydroxybutylamine de formule (I) caractérisé en ce que

(a) on traite le 3,4-dichlorophénylacétonitrile de formule (II):

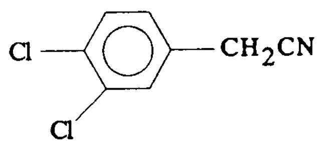

(II)

avec un haloacétate alcalin dans l'ammoniac liquide ou dans un solvant aprotique polaire en présence d'une base forte à une température de -40°C à +25°C,

(b) on traite l'acide 3-cyano-3-(3,4-dichlorophényl)propionique ainsi obtenu racémique de formule (III):

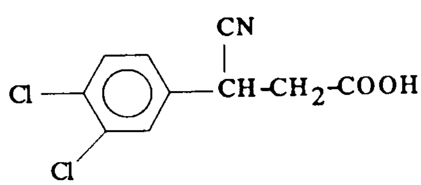

(III)

avec la D-(-)-N-méthylglucamine pour obtenir la cristallisation de la totalité de l'acide (III) sous la forme de sel de D(-)-N-méthylglucamine de l'acide lévogyre;

(c) on traite ledit sel avec un acide fort ; et

(d) on soumet l'acide (-)-3-cyano-3-(3,4-dichlorophényl)propionique ainsi libéré de formule (IV):

formule (IV) :

(-) 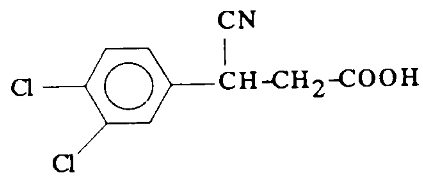 (IV)

à une réduction énantioconservatrice avec un borane conduisant au dérivé (I).

Plus particulièrement, dans l'étape (a) on utilise un haloacétate alcalin, tel que le chloroacétate de sodium ou de potassium ou le bromoacétate de sodium ou de potassium en présence d'une base forte telle que l'amidure de sodium, le *tert*-butylate de sodium, l'éthylate de sodium. Comme solvant on peut utiliser l'ammoniac liquide à basse température (-40°C à -30°C) ou un solvant aprotique polaire, inerte dans les conditions réactionnelles, comme le diméthylsulfoxyde, le N,N-diméthylformamide. Le cyanoacide de formule (III) ainsi obtenu après une réaction de 4-5 heures, est isolé par traitement avec de l'eau ou des mélanges eau/éther, par exemple l'éther isopropylique. Il peut être transformé en un de ses sels.

L'étape (b) peut être effectuée sur le cyanoacide (III) isolé ou bien *in situ* directement après l'étape (a) dans un solvant tel qu'un alcool, de préférence l'éthanol. Le sel de D(-)-glucamine de l'acide (-)-3-cyano-3-(3,4-dichlorophényl)propionique cristallise directement et peut être isolé.

L'acide lévogyre est libéré de son sel, selon l'étape (c), par traitement avec un acide fort et isolé par extraction avec un solvant approprié, tel que le dichlorométhane, le dichloroéthane, le 1,1,1-trichloroéthane. L'acide (IV) peut être transformé en un de ses sels.

Dans l'étape (d), la réduction énantioconservatrice avec un borane, tel que le $BH_3$, le $B_2H_6$ éventuellement sous forme d'un complexe avec le tétrahydrofurane ou le diméthylsulfure, est effectuée à la température ambiante dans un solvant du type éther, tel que le dioxane ou le tétrahydrofurane. L'aminoalcool (I) est isolé, après avoir détruit l'excès de borane et évaporé le solvant, par l'élimination des produits secondaires par traitements successifs avec un acide puis avec une base, suivie d'une extraction avec un solvant approprié, tel que le dichlorométhane, le dichloroéthane, le 1,1,1-trichloroéthane.

L'acide 3-cyano-3-(3,4-dichlorophényl)propionique (III) est préparé par action du chloroacétate de sodium sur le 3,4-dichlorophénylacétonitrile (II) par exemple, dans l'ammoniac liquide en présence d'amidure de sodium selon la technique de A.G. CHIGAREV et D.V. IOFFE, Zh. Org. Khim. 3, 85-8, (1967) ou d'une autre base très forte comme le tertiobutylate de sodium ou de potassium dans l'ammoniac liquide à -33°C ou dans le diméthylsulfoxyde anhydre à température ambiante. Le rendement en acide 3-cyano-3-(3,4-dichlorophényl)propionique isolé est de 74 à 78 %, mais il est encore meilleur si le produit n'est pas isolé car il suffit de faire agir la D-(-)-N-méthylglucamine, produit industriel peu coûteux, obtenu à partir de D-glucose et de méthylamine (KARRER, HERKENRATH - Helv. Chim. Acta, *20*, 37 (1937)), pour faire cristalliser la totalité du cyanoacide racémique (III) sous la forme de sel de l'acide lévogyre. Le rendement est excellent, puisqu'il est de 190 % par rapport à l'énantiomère lévogyre contenu dans le racémique (III). Le solvant de cristallisation peut être le méthanol, l'éthanol, le Cellosolve® ou tout autre solvant adapté. La température de dédoublement est comprise entre le point d'ébullition du solvant et 0°C. La N-méthylglucamine doit être présente en quantité au moins stoechiométrique. On l'utilise de préférence en léger excès.

Après libération de l'acide 3-cyano-3-(3,4-dichlorophényl)propionique de son sel avec la D-(-)-N-méthylglucamine par l'action d'un acide fort comme l'acide chlorhydrique, l'acide oxalique ou une résine échangeuse d'ion type sulfonique, il est réduit de façon conservatrice (pureté énantiomérique 99 %) par le borane.

Le rendement de cette double réduction est d'au moins 70 %.

Le borane peut être mis en oeuvre sous forme de son dimère $B_2H_6$, mais de préférence sous une forme plus manipulable telle que la forme de complexe avec le tétrahydrofurane ou avec le diméthylsulfure commercialisé sous le nom de B.M.S.

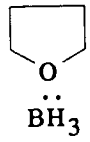 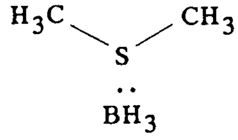

La transformation asymétrique de second ordre selon l'étape (b) du procédé de la présente invention est surprenant et constitue un autre objet de la présente invention, qui concerne ainsi, selon un autre de ses aspects, un procédé pour

la préparation de l'acide (-)-3-cyano-3-(3,4-dichlorophényl)propionique caractérisé en ce que on traite l'acide 3 cyano-3-(3,4-dichlorophényl)propionique racémique avec la D-(-)-N-et on traite le sel de D-(-)-N-méthylglucamine de l'acide (-)-3-cyano-3-(3,4-dichlorophényl)propionique ainsi obtenu avec un acide fort.

L'acide 3-cyano-3-(3,4-dichlorophényl)propionique (III) et ses sels, son isomère (-) de formule (IV) et ses sels sont des produits nouveaux et représentent un autre aspect de la présente invention.

Plus particulièrement, le sel de D-(-)-N-méthylglucamine de l'acide (-)-3-cyano-3-(3,4-dichlorophényl)propionique constitue un aspect ultérieur de la présente invention.

Le composé (I) tel qu'obtenu par le nouveau procédé selon l'invention peut avantageusement être utilisé pour la synthèse stéréosélective d'arylalkylamines optiquement pures, antagonistes des récepteurs des neurokinines.

En particulier, le composé (I) peut être utilisé pour la préparation des arylalkylamines décrites dans les demandes EP 0 428 434, 0 474 561, 0 515 240, et 0 559 538 selon le schéma de synthèse général illustré par le schéma (1) ci-dessous, dans lequel les substituants B et D représentent l'ensemble des substituants du cycle aminé des arylalkylamines décrites dans lesdites demandes de brevet, les substituants W, T et Z sont tels que décrits dans ces demandes et Ar' représente un groupe dichlorophényle.

SCHEMA 1

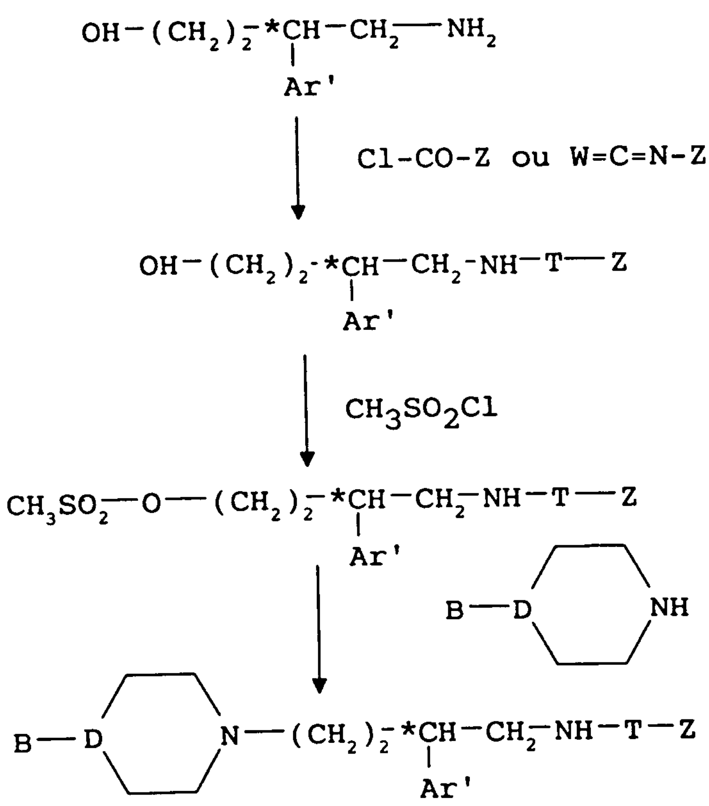


L'astérisque "*" signifie que l'atome de carbone ainsi marqué est de configuration (+) ou (-) déterminée.

De préférence, on utilisera le composé de formule (I) tel qu'obtenu selon le procédé de l'invention pour la préparation d'arylalkylamines optiquement pures de formule (VI)

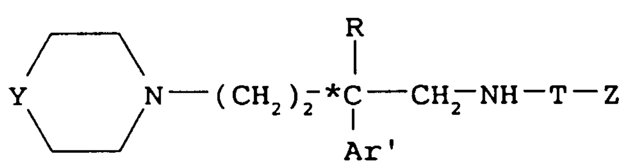

(VI)

dans laquelle:

- Y représente - soit un groupe Cy-N dans lequel

  . Cy représente un phényle non substitué ou substitué une ou plusieurs fois par l'un des substituants choisis parmi : l'hydrogène, un atome d'halogène, un hydroxyle, un alcoxy en $C_1$-$C_4$, un alkyle en $C_1$-$C_4$, un trifluorométhyle, lesdits substituants étant identiques ou différents, un groupe cycloalkyle en $C_3$-$C_7$; un groupe pyrimidinyle ou un groupe pyridyle;

  - soit un groupe

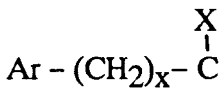

  dans lequel

  . Ar représente un phényle non substitué ou substitué une ou plusieurs fois par l'un des substituants choisi parmi : l'hydrogène, un atome d'halogène, un hydroxyle, un alcoxy en $C_1$-$C_4$, un trifluorométhyle, un alkyle en $C_1$-$C_4$, lesdits substituants étant identiques ou différents ; un groupe pyridyle ; un groupe thiényle;

  . x est zéro ou un;

  . X représente un hydroxyle, un alcoxy en $C_1$-$C_4$ ; un hydroxyalkyle dans lequel l'alkyle est en $C_1$-$C_3$ ; un acyloxy en $C_1$-$C_4$ ; un phénacyloxy ; un carboxy ; un carbalcoxy en $C_1$-$C_4$ ; un cyano ; un aminoalkylène dans lequel l'alkylène est en $C_1$-$C_3$ ; un groupe-N-$(X_1)_2$ dans lequel les groupes $X_1$ représentent indépendamment l'hydrogène, un alkyle en $C_1$-$C_4$ ; un groupe -NH-CO-Alk dans lequel Alk représentent un alkyle en $C_1$-$C_6$ ; un groupe -$Alk_1$-NH-CO-$Alk'_1$ dans lequel $Alk_1$ est un alkylène en $C_1$-$C_3$ et $Alk'_1$ est un alkyle en $C_1$-$C_3$; un acyle en $C_1$-$C_4$ ; un groupe -S-$X_2$ dans lequel $X_2$ représente l'hydrogène ou un groupe alkyle en $C_1$-$C_4$; ou bien X forme avec l'atome de carbone auquel il est lié et avec l'atome de carbone voisin dans l'hétérocycle une double liaison;

- Ar' représente un groupe dichlorophényle,
- R représente l'hydrogène,
- T représente un groupe choisi parmi

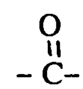 et 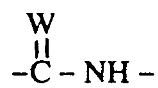

W étant un atome d'oxygène ou de soufre, et

- Z représente soit l'hydrogène, soit M ou OM lorsque T représente le groupe

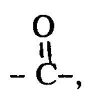,

soit M lorsque T représente le groupe

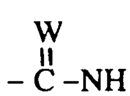 ;

M représente un alkyle en $C_1$-$C_6$ ; un phénylalkyle dans lequel l'alkyle est en $C_1$-$C_3$, éventuellement substitué sur le cycle aromatique par un halogène, un trifluorométhyle, un alkyle en $C_1$-$C_4$, un hydroxyle, un alcoxy en $C_1$-$C_4$,; un pyridylalkyle dans lequel l'alkyle est en $C_1$-$C_3$ ; un naphtylalkyle dans lequel l'alkyle est en $C_1$-$C_3$, éventuellement substitué sur le cycle naphtyle par un halogène, un trifluorométhyle, un alkyle en $C_1$-$C_4$, un hydroxyle, un alcoxy en $C_1$-$C_4$ ; un pyridylthioalkyle dans lequel l'alkyle est en $C_1$-$C_3$ ; un styryle; un groupe aromatique ou hétéroaromatique

mono-, di- ou tricyclique éventuellement substitué ;
ou un de ses sels avec des acides minéraux ou organiques.

Les composés de formule (VI) décrits dans la demande EP 0 474 561, sont préparés selon le schéma 1 mentionné plus haut dans lequel B - D est représenté dans la formule (VI) par Y.

En particulier, le composé de formule (I) tel qu'obtenu par le procédé selon l'invention peut-être avantageusement utilisé pour la préparation du chlorhydrate ou du méthanesulfonate de (-) N-méthyl N-[(phényl-4 acétylamino-4 pipéridinyl)-4 (dichloro-3,4 phényl)-2 butyl] benzamide.

On peut également utiliser le composé de formule (I) tel qu'obtenu par le procédé selon l'invention pour la préparation d'amides basiques quaternaires optiquement pures de formule (VII)

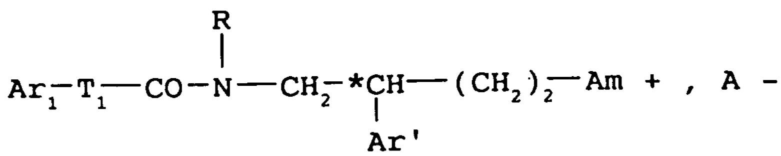

(VII)

dans laquelle

- $Ar_1$ représente un groupe aromatique ou hétéroaromatique mono-, di- ou tricyclique éventuellement substitué;
- $T_1$ représente une liaison directe, un groupe hydroxyméthylène, un groupe alcoxyméthylène dont le groupe alcoxy est en $C_1$–$C_4$ ou un groupe alkylène en $C_1$-$C_5$
- Ar' représente un groupe dichlorophényle;
- R représente l'hydrogène;
- $Am^{\oplus}$ représente le radical

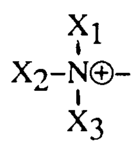

dans lequel $X_1$, $X_2$, $X_3$, ensemble et avec l'atome d'azote auquel ils sont liés, forment un système azabicyclique ou azatricyclique éventuellement substitué par un group phényle ou benzyle ;
- $A^{\ominus}$ est un anion pharmaceutiquement acceptable.

Les composés de formule (VII) sont préparés par un procédé consistant :

- à faire réagir un composé de formule (I) tel qu'obtenu selon le procédé de l'invention avec un composé de formule

$$Ar_1\text{-}T_1\text{-}CO\text{-}OH$$

dans laquelle $T_1$ et $Ar_1$ sont tels que définis ci-dessus et,
- à faire réagir le composé obtenu, de formule

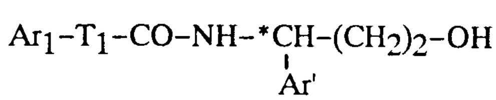

dans laquelle Ar' est tel que défini ci-dessus avec un composé de formule G-Cl, dans laquelle G représente un groupe partant tel que le groupe mésyle ou benzènesulfonyle puis,

- à faire réagir le composé obtenu de formule:

$$\begin{array}{l} Ar_1-T_1-CO-NH-{}^*\underset{\displaystyle Ar'}{\underset{|}{CH}}-(CH_2)_2-OG \end{array}$$

avec une amine tertiaire de formule :

$$\begin{array}{rl} & X_1 \\ & | \\ X_2- & N \\ & | \\ & X_3 \end{array}$$

dans laquelle $X_1$, $X_2$, $X_3$ sont tels que définis ci-dessus, dans un solvant organique à une température comprise entre la température ambiante et 120°C, et

- à isoler le produit ainsi obtenu ou bien, éventuellement, on échange l'anion méthanesulfonate du sel quaternaire ainsi obtenu avec un autre anion pharmaceutiquement acceptable.

En particulier, le composé de formule (I) tel qu'obtenu selon le procédé de l'invention sera avantageusement utilisé pour la préparation du chlorure de (+) 1-[2-[3-(3,4-dichlorophényl)-1-[(3-isopropoxyphényl)acétyl]-3-pipéridinyl]éthyl]-4-phényl-1-azoniabicyclo[2.2.2)octane.

Les exemples suivants illustrent l'invention sans toutefois la limiter.

EXEMPLE 1

Acide (±)-3-cyano-3-(3,4-dichlorophényl)propionique (III).

On fait réagir pendant 5 heures à température ambiante un mélange de 18,6 g (0,10 mole) de 3,4-dichlorophénylacétonitrile et de 12 g (1,03 moles) de chloroacétate de sodium sec dans 150 ml de diméthylsulfoxyde sec, en présence de 10,5 g (1,05 moles) de *tert*-butylate de sodium. Après réaction, le mélange réactionnel est coulé sur 1 litre d'eau glacée et est acidifié à pH < 3 par de l'acide chlorhydrique. Le cyanoacide est extrait par de l'acétate d'éthyle, qui est lavé jusqu'à pH > 3, séché sur sulfate de magnésium et concentré à sec. Le résidu est concrétisé dans du 1,2-dichloroéthane. On obtient 16,1 g de composé (III) attendu et caractérisé par RMN du proton.

EXEMPLE 2

Acide (±)-3-cyano-3-(3,4-dichlorophényl)propionique (III).

On fait réagir pendant 4 heures à -33°C un mélange de 93 g (0,50 mole) de 3,4-dichlorophénylacétonitrile et de 64 g (0,55 mole) de chloroacétate de sodium dans 500 ml d'ammoniac liquide, en présence de 21 g (0,54 mole) d'amidure de sodium. Après évaporation de l'ammoniac, le résidu est repris par de l'eau, puis par de l'éther isopropylique et est acidifié à pH < 3 par de l'acide chlorhydrique. La phase organique est lavée à l'eau jusqu'à pH > 3, séparée par décantation, séchée sur sulfate de magnésium et concentrée à sec. Le résidu est concrétisé dans le toluène et caractérisé par RMN du proton. F = 106°C

EXEMPLE 3

Acide (±)-3-cyano-3-(3,4-dichlorophényl)propionique (III).

On fait réagir pendant 4 heures à -33°C un mélange de 186 g (1,00 mole) de 3,4-dichlorophénylacétonitrile, 126 g (1,05 moles) de chloroacétate de sodium et 105 g (1,05 moles) de *tert*-butylate de sodium dans 1 litre d'ammoniac liquide. Après réaction l'ammoniac est évaporé, le résidu est repris par 500 ml d'eau glacée, puis par 500 ml d'éther isopropylique et est acidifié à pH < 3 par de l'acide chlorhydrique. La phase aqueuse est écartée, la phase organique est lavée à l'eau jusqu'à pH > 3, séparée par décantation, séchée sur sulfate de magnésium et concentrée sous vide. Le résidu est concrétisé dans 250 ml de toluène, le cyanoacide est filtré et séché à 50°C sous vide de pompe à palettes. On obtient 190 g (rendement 78 %) d'acide (±)-3-cyano-3-(3,4-dichlorophényl)propionique attendu. F = 104°C.

Le produit est caractérisé par RMN du proton à 200 MHz dans le DMSO:

- massif entre 2,85 et 3,1 ppm, 2 protons : $-CH_2-$

- signal complexe à 4,5 ppm, 1 proton -CH-
- protons aromatiques entre 7,4 et 7,75 ppm, 3 protons
- un proton acide à 12,8 ppm

EXEMPLE 4

Acide (-)-3-cyano-3-(3,4-dichlorophényl)propionique (IV).

On fait réagir pendant 4 heures à -33°C un mélange de 186 g (1,00 mole) de 3,4-dichlorophénylacétonitrile, 126 g (1,05 moles) de chloroacétate de sodium et 105 g (1,05 moles) de *tert*-butylate de sodium dans 1 litre d'ammoniac liquide. Après réaction l'ammoniac est évaporé, le résidu est repris par 500 ml d'eau glacée, puis par 500 ml d'éther isopropylique et est acidifié à pH < 3 par de l'acide chlorhydrique. La phase aqueuse est écartée, la phase organique est lavée à l'eau jusqu'à pH > 3, séparée par décantation,séchée sur sulfate de magnésium et concentrée sous vide. Le concentrat est redissous dans 2 litres d'éthanol absolu, chauffé et additionné de 292 g de D-(-)-N-méthylglucamine. Après cristallisation, le produit est filtré, rincé à l'éthanol et séché sous vide. On obtient 396 g d'acide (-)-3-cyano-3-(3,4-dichlorophényl)propionique, sel de N-méthylglucamine,

$[\alpha]_D^{25}$ = -14,7° (C = 1, $CH_3OH$).

Le rendement est de 91 % par rapport au 3,4-dichlorophénylacétonitrile.

Le sel obtenu est dissout dans 900 ml d'acide chlorhydrique 1N et extrait par 2 litres de dichlorométhane. La phase organique est lavée à l'eau, séparée par décantation, séchée sur sulfate de sodium et concentrée. Le produit est concrétisé dans 500 ml de cyclohexane. On obtient ainsi 187 g de produit attendu. Le rendement est de 76,5 % par rapport au 3,4-dichlorophénylacétonitrile. F = 98°C.

$[\alpha]_D^{25}$ = -8,6° (C = 1, $CH_3OH$).

Pureté énantiomérique par HPLC: 99 %.

RMN du proton à 200 MHz dans le DMSO : même spectre que le racémique.

EXEMPLE 5

(+)-2-(3,4-dichlorophényl)-4-hydroxybutylamine (I).

A une solution de 244 g (1 mole) d'acide (-)-3-cyano-3-(3,4-dichlorophényl)propionique dans 500 ml de THF refroidie à 0°C, on ajoute 350 ml d'une solution 1 molaire de $BH_3$ dans le THF. Quand le dégagement d'hydrogène a cessé, 650 ml de la solution de borane sont ajoutés à 20°C, puis 1000 ml à 40°C. Lorsque la réaction est terminée, l'excès de borane est détruit par addition de méthanol et le mélange réactionnel est concentré à sec. Le concentrat est dissous dans 500 ml d'eau, acidifié par de l'acide chlorhydrique et lavé par deux fois avec 250 ml de toluène. La phase aqueuse est alcalinisée par de l'hydroxyde de sodium et extraite par deux fois 400 ml de dichlorométhane. La phase organique est lavée à l'eau, séparée par décantation, séchée sur sulfate de magnésium et concentrée sous vide. On obtient 159 g (rendement 68 %) de produit attendu. Pureté chirale par HPLC : 99 %.

Le produit est caractérisé par RMN du proton dans le $CDCl_3$ à 200 MHz:

- un massif à 1,8 ppm 2H
- un singulet à 2,4 ppm 3H
- un massif entre 2,65 et 2,9 ppm 3H
- un massif entre 3,35 et 3,6 ppm 2
- protons aromatiques entre 6,95 et 7,35 ppm 3H

$[\alpha]_D^{25}$ = +9,8° (C = 1, MeOH).

F = 80-81°C.

EXEMPLE 6

(+)-2-(3,4-Dichlorophényl)-4-hydroxybutylamine (I).

On opère selon l'EXEMPLE 5, mais en utilisant du borane diméthylsulfure, en diluant avec 750 ml de tétrahydrofurane et en opérant à 20°C puis 50°C. On obtient le même produit, avec le même rendement et les mêmes caractéristiques.

## Revendications

1. Procédé pour la préparation de la (+)-2-(3,4-dichlorophényl)-4-hydroxybutylamine de formule (I):

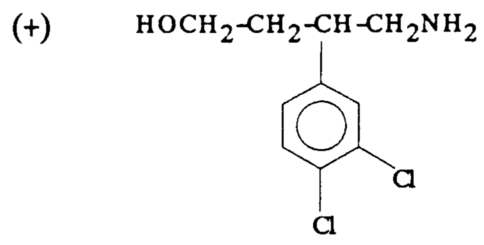

(I)

caractérisé en ce que

(a) on traite le 3,4-dichlorophénylacétonitrile de formule (II) :

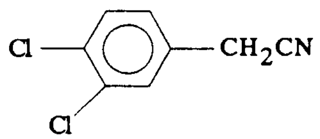

(II)

avec un haloacétate alcalin dans l'ammoniac liquide ou dans un solvant aprotique polaire en présence d'une base forte à une température de -40°C à +25°C,

(b) on traite l'acide 3-cyano-3-(3,4-dichlorophényl)propionique ainsi obtenu racémique de formule (III) :

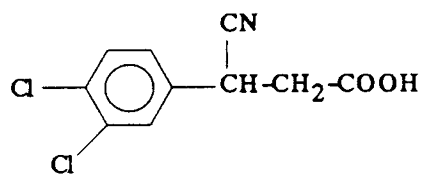

(III)

avec la D-(-)-N-méthylglucamine pour obtenir la cristallisation de la totalité de l'acide (III) sous la forme de sel de D(-)-N-méthylglucamine de l'acide lévogyre;

(c) on traite ledit sel avec un acide fort ; et

(d) on soumet l'acide (-)-3-cyano-3-(3,4-dichlorophényl)propionique ainsi libéré de formule (IV) :

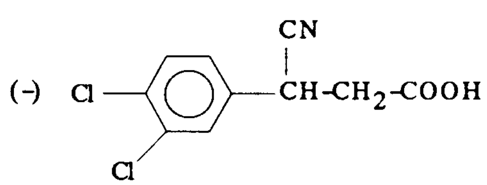

(IV)

à une réduction énantioconservatrice avec un borane.

2. Procédé selon la revendication 1, caractérisé en ce que l'étape (a) est conduite dans l'ammoniac liquide à une température de -40 à -30°C.

3. Procédé selon la revendication 2, caractérisé en ce que l'étape (a) est conduite en présence d'amidure de sodium ou de *tert*-butylate de sodium.

4. Procédé selon la revendication 1, caractérisé en ce que l'étape (a) est conduite dans un solvant aprotique polaire, inerte dans les conditions de réaction.

5. Procédé selon l'une quelconque des revendications 1 à 4, caractérisé en ce que comme haloacétate alcalin on utilise le chloroacétate de sodium.

6. Procédé pour la préparation de l'acide (-)-3-cyano-3-(3,4-dichlorophényl)propionique caractérisé en ce que on traite l'acide 3-cyano-3-(3,4-dichlorophényl)propionique racémique avec la D-(-)-N-méthylglucamine et on traite le sel de D-(-)-N-méthylglucamine de l'acide (-)-3-cyano-3-(3,4-dichlorophényl)propionique ainsi obtenu avec un acide fort.

7. Acide 3-cyano-3-(3,4-dichlorophényl)propionique de formule :

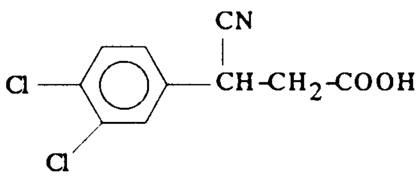

et ses sels.

8. Acide (-)-3-cyano-3-(3,4-dichlorophényl)propionique et ses sels.

9. Sel de D-(-)-N-méthylglucamine de l'acide (-)-3-cyano-3-(3,4-dichlorophényl)propionique.

**Claims**

1. Process for the preparation of (+)-2-(3,4-dichlorophenyl)-4-hydroxybutylamine of formula (I):

(+)

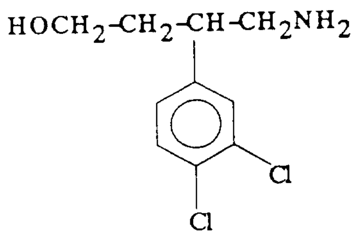

(I)

characterized in that

(a) the 3,4-dichlorophenylacetonitrile of formula (II):

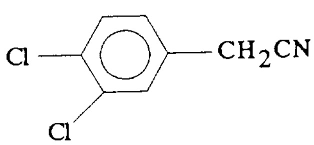

(II)

is treated with an alkali metal halogenoacetate in liquid ammonia or in a polar aprotic solvent, in the presence of a strong base, at a temperature of -40°C to +25°C;

(b) the resulting racemic 3-cyano-3-(3,4-dichlorophenyl)propionic acid of formula (III):

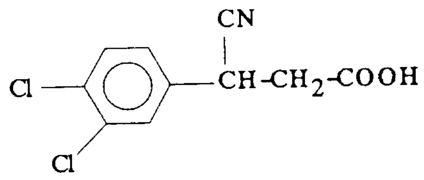

(III)

is treated with D-(-)-N-methylglucamine in order to crystallize all the acid (III) in the form of the D-(-)-N-methylglucamine salt of the levorotatory acid;

(c) said salt is treated with a strong acid; and

(d) the thus freed (-)-3-cyano-3-(3,4-dichlorophenyl)propionic acid of formula (IV):

(-) 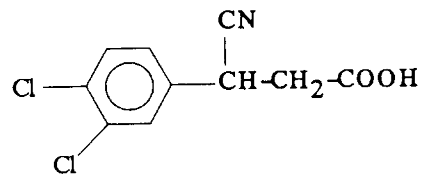

(IV)

is subjected to an enantioconservative reduction with a borane.

**2.** Process according to claim 1, characterized in that step (a) is carried out in liquid ammonia at a temperature of -40 to -30°C.

**3.** Process according to claim 2, characterized in that step (a) is carried out in the presence of sodium amide or sodium *tert*-butylate.

**4.** Process according to claim 1, characterized in that step (a) is carried out in a polar aprotic solvent which is inert under the reaction conditions.

**5.** Process according to any one of claims 1 to 4, characterized in that sodium chloroacetate is used as the alkali metal halogenoacetate.

**6.** Process for the preparation of (-)-3-cyano-3-(3,4-dichlorophenyl)propionic acid, characterized in that the racemic 3-cyano-3-(3,4-dichlorophenyl)propionic acid is treated with D-(-)-N-methylglucamine and the resulting D-(-)-N-methylglucamine salt of (-)-3-cyano-3-(3,4-dichlorophenyl)propionic acid is treated with a strong acid.

**7.** 3-Cyano-3-(3,4-dichlorophenyl)propionic acid of the formula

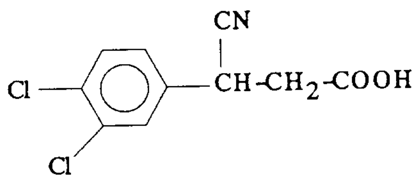

and its salts.

**8.** (-)-3-Cyano-3-(3,4-dichlorophenyl)propionic acid and its salts.

**9.** D-(-)-N-methylglucamine salt of (-)-3-cyano-3-(3,4-dichlorophenyl)propionic acid.

**Patentansprüche**

1. Verfahren zur Herstellung von (+)-2-(3,4-Dichlorphenyl)-4-hydroxybutylamin der Formel (I):

(+) 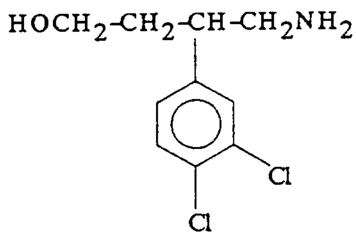 (I)

gekennzeichnet durch folgende Schritte:

(a) Behandeln des 3,4-Dichlorphenylacetonitrils der

Formel (II):

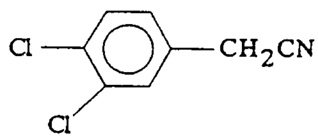 (II)

mit einem Alkalihalogenacetat in flüssigem Ammoniak oder in einem aprotischen polaren Lösungsmittel in Gegenwart einer starken Base bei einer Temperatur von -40 bis +25 °C,

(b) Behandeln der so erhaltenen racemischen 3-Cyano--3-(3,4-dichlorphenyl)-propionsäure der Formel (III):

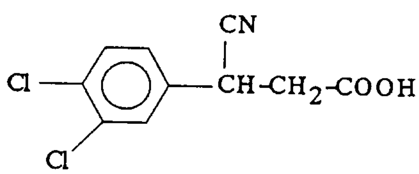 (III)

mit D-(-)-N-Methylglucamin zur Erzielung der Kristallisation der gesamten Säure (III) in Form des Salzes aus D(-)-N-Methylglucamin und der linksdrehenden Säure,

(c) Behandeln des Salzes mit einer starken Säure und

(d) Durchführen einer enantiokonservativen Reduktion der so freigesetzten (-)-3-Cyano-3-(3,4-dichlorphenyl)-propionsäure der Formel (IV):

(-) 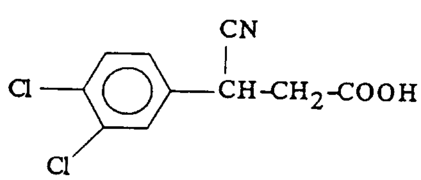 (IV)

mit einem Boran.

2. Verfahren nach Anspruch 1, dadurch gekennzeichnet, daß Schritt (a) in flüssigem Ammoniak bei einer Temperatur von -40 bis -30 °C durchgeführt wird.

3. Verfahren nach Anspruch 2, dadurch gekennzeichnet, daß Schritt (a) in Gegenwart von Natriumamid oder Natrium-*tert.*-butylat durchgeführt wird.

4. Verfahren nach Anspruch 1, dadurch gekennzeichnet, daß Schritt (a) in einem polaren aprotischen Lösungsmittel durchgeführt wird, das unter den Reaktionsbedingungen inert ist.

5. Verfahren nach einem der Ansprüche 1 bis 4, dadurch gekennzeichnet, daß Natriumchloracetat als Alkalihalogenacetat verwendet wird.

6. Verfahren zur Herstellung der (-)-3-Cyano-3-(3,4-dichlorphenyl)-propionsäure, dadurch gekennzeichnet, daß die racemische 3-Cyano-3-(3,4-dichlorphenyl)-propionsäure mit D-(-)-N-Methylglucamin und das so erhaltene Salz aus D(-)-N-Methylglucamin und der (-)-3-Cyano-3-(3,4-dichlorphenyl)-propionsäure mit einer starken Säure behandelt wird.

7. 3-Cyano-3-(3,4-dichlorphenyl)-propionsäure der Formel

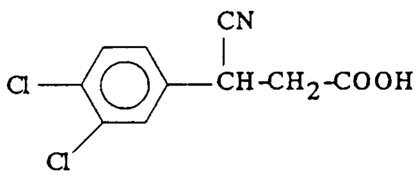

und ihre Salze.

8. (-)-3-Cyano-3-(3,4-dichlorphenyl)-propionsäure und ihre Salze.

9. Salz aus D-(-)-N-Methylglucamin und der (-)-3-Cyano-3-(3,4-dichlorphenyl)propionsäure.